# EUROPEAN PATENT SPECIFICATION

(11) **EP 3 031 932 B1**
(45) Date of publication and mention of the grant of the patent: **21.08.2019**
(21) Application number: 14004143.5
(22) Date of filing: 09.12.2014
(51) Int. Cl.: C12R 1/89, C12N 1/00, C12N 1/04, C12N 1/12, C12P 7/64

(54) **A new microalgae chlorella for production of vegetal oil for biodiesel and cogeneration power units**
Neue Mikroalge Chlorella zur Herstellung von Pflanzenöl für Biodiesel und Wärmekrafteinheiten
Nouvelle microalgue de type chlorelle pour la production de biodiesel et d'huile végétale et unités de cogénération d'énergie

(43) Date of publication of application: 15.06.2016
(73) Proprietor: Magri, Michael, 41050 Formigine (Modena) (IT)
(72) Inventor: Magri, Michael, 41050 Formigine (Modena) (IT)
(74) Representative: Bergamini, Silvio

(56) References cited:
- WO-A1-2014/083534
- CN-A- 103 114 041
- ALESSIA GUCCIONE ET AL: "Chlorella for protein and biofuels: from strain selection to outdoor cultivation in a Green Wall Panel photobioreactor", BIOTECHNOLOGY FOR BIOFUELS, BIOMED CENTRAL LTD, GB, vol. 7, no. 1, 7 June 2014 (2014-06-07), page 84, XP021188565, ISSN: 1754-6834, DOI: 10.1186/1754-6834-7-84

## Description

### Field of the invention.

The invention relates to the field of renewable bioenergy, and particularly addresses a novel method of producing biodiesel through two-stage culture of Chlorella Vulgaris.

### Background art.

Industrial utilization of microorganisms, cyanobacteria and particularly microalgae is known to be limited by the high cost of culture systems.

Excepting a few particular cases (production of high-value products such as labeled microalgae), the industrial production of microalgae and derivatives requires plants that can produce hundreds or thousands of kilograms of biomass per year.

In particular fields, such as feeds and biofuels, the required throughput is hundreds or thousands of tons of biomass per year.

Since the throughput of culture systems for phototrophic microorganisms, or photobioreactors, rarely exceeds 2 grams per liter per day, industrial plants for microalgal growth must use culture systems that can contain tens or hundreds of cubic meters culture.

Problems arise from technical and biological constraints, including the need for microalgae that can considerably exceed 2 grams/liter.

The term biodiesel is particularly intended to designate long chains of mono alkyl esters obtained from fatty acids through a trans-esterification process.

At present, microalgae are among the most interesting and sustainable sources for biodiesel production.

Two large-scale cultivation types exist: phototrophic cultivation and heterotrophic fermentation.

Phototrophic cultivation consists in supplying light and CO₂ to the microalgae.

Due to the mutual shadowing effect caused by the increased cell concentration, lipid synthesis is limited.

Under heterotrophic conditions, provided that the proper amount of organic carbon to be supplied is found, large amounts of lipids may be synthesized.

### Brief description of the drawings

FIG. 1 is a growth table for a microalga Chlorella Vulgaris;
FIG. 2 is a growth table for a microalga Chlorella Vulgaris Mod;
FIG. 3 is a photographic representation of the chloroplast during the growth process.

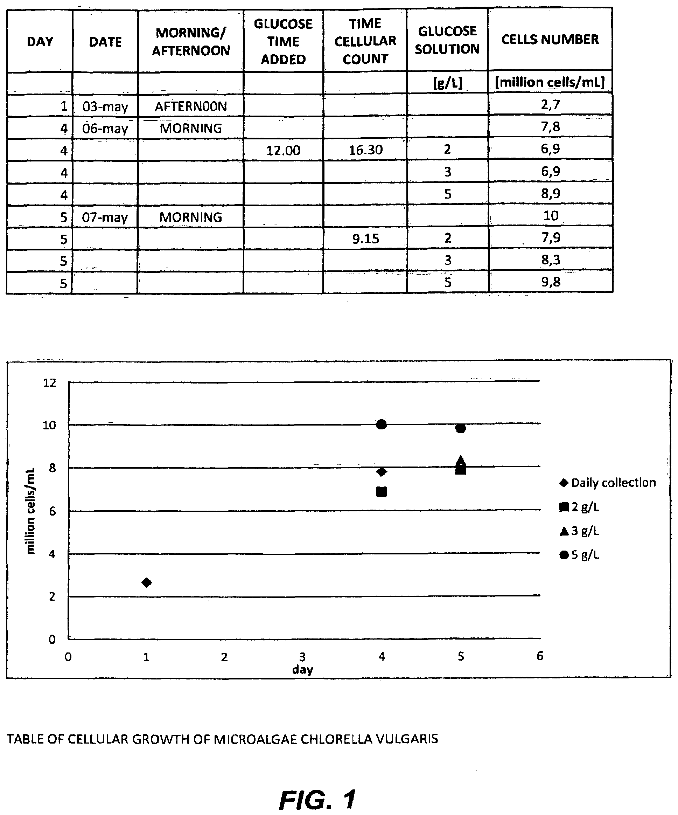

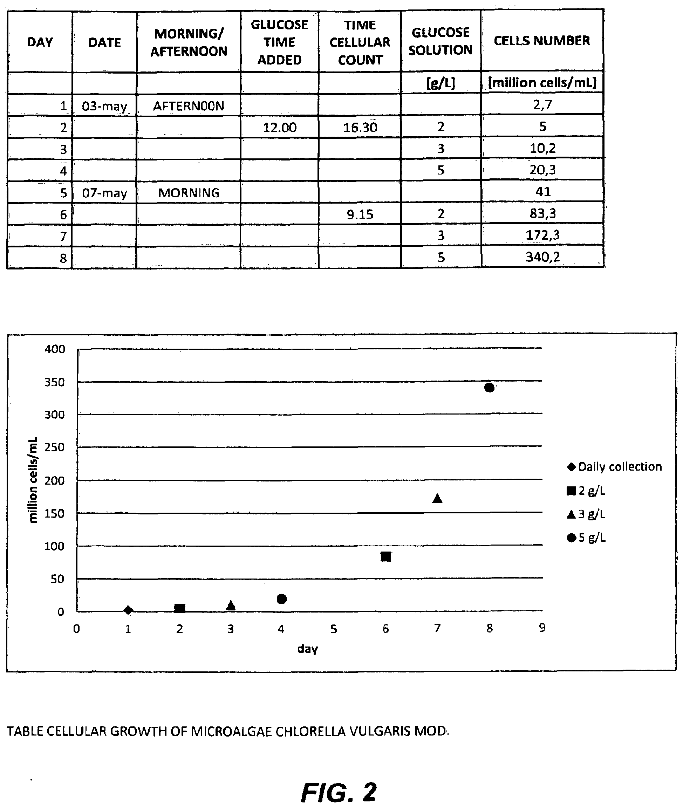

Referring to the figures, the microalga of the present invention exhibit a much higher lipid productivity than Chlorella Vulgaris as shown in the table of Figure 1 and Chlorella Vulgaris defined Mod for simplicity in Figure 2. At the same date and time a comparative test was started under the same test conditions and, at day 8, Chlorella Vulgaris Mod had its cells increased to 340.2 millions, whereas Chlorella Vulgaris only increased by 9.8 millions.

### Detailed description of the present invention

The microalga of the invention is derived from Chlorella Vulgaris and can produce light aliphatic hydrocarbons having 16-26 carbons, and have the peculiar property of being able to grow under both phototrophic cultivation and heterotrophic fermentation conditions, unlike many other types of microalgae which only grow under one of the two conditions.

Sustainability of the process relies on various factors: phototrophic cultivation growth must use CO₂ derived from combustion, engine or methane furnace fumes, light must be sunlight and cannot be artificial light, water must be well water and not come from the water supply network.

If the algae were only phototrophic, at night times they would have to be lighted by artificial light, at high costs, whereas since they are also heterotrophic, they are fed with by-products having glucose therein, and can thus be fermented in tanks.

Full development of this process requires high-throughput and high lipid-content microalgae, therefore Chlorella Vulgaris was selected, for maximized utilization of its potential.

This study has been conducted with the aim of increasing its natural productivity and without changing it genetically the method as described below has been found: some algal species are richer in advantageous compounds for nutraceutical and pharmaceutical industries, whereas other species are richer in vegetable oil and hence are interesting for biofuels.

The data that allows selection of the various strains for biodiesel production are the achievable biomass concentration and the oil content that can be produced by microalgae, on a dry weight basis.

Therefore, the selection of the algal strain will be made according to the most useful characteristics for the desired final production.

On the other hand, for algae to accumulate oil, they must be exposed to stress conditions, to such an extent as to induce emergency behaviors.

Particularly, the carbon to nitrogen ratio must be increased, i.e. the biomass must be deprived of nitrogenated nutrients or the system muse be deprived of phosphorus.

One of the most interesting characteristics, in addition to the concentration and percent of lipids that will eventually become fuel oil, is reproductivity.

The effect of light radiation is important for both algal growth and oil production, and for this reason photobioreactors were used which could differentiate light absorption according to the most useful parameters for growth.

This novel technology, that has never been used in any existing plant, affords considerable throughput increase. Nevertheless, for optimized light absorption throughout the algal mass, an alga of the same strain with improved characteristics had to be developed.

This is because the innermost algal layers in the photobioreactors hardly receive an adequate stream of photons.

This third-generation algal strain resulted from many years of research.

Thus, for each algal strain, a corresponding third-generation "collaborative alga", i.e. the twin of the naturally occurring (i.e. wild) alga, was developed by natural selection.

For a better understanding of the advantage of this innovation, it should be noted that light is captured in the chloroplast, which is somewhat the mouth, whereas lipid synthesis and storage occurs in the cytoplasm, acting as a stomach.

The modification basically consists in reducing the size of the chloroplast, such that it cannot absorb excess light, and in increasing the volume of the cytoplasm, for storage of a greater amount of lipids.

In short, a large mouth would cause indigestion, with regurgitation and waste of food, whereas a small mouth would allow the stomach to use the whole ingested food, some of it being also left for the other algae, such that nothing would be wasted.

The size of the chloroplast is reduced by immersion in a tank containing a water-soluble conductive gel that allows the microalgae to survive, the tank being surrounded by low-frequency ultrasonic transducers with adjustable frequency and power.

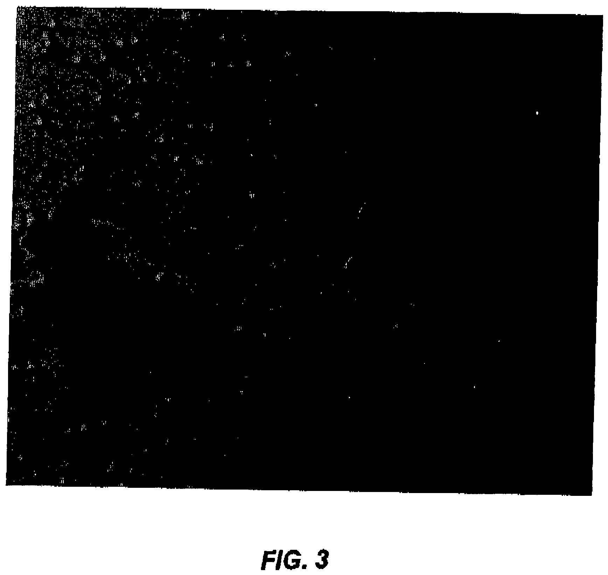

The gel affords greatly improved ultrasound transmission by the transducers as compared with water, and also maintains the microalgae still during such transmission. The particular transmitted frequency and power prevent development of the chloroplast during initial growth and allow it to maintain the same thickness even during growth, as shown in Figure 3.

The invention has been found to fulfill the intended objects.

The invention so conceived is susceptible to changes and variants within the inventive concept.

Also, all the details may be replaced by other technical equivalent elements.

In its practical implementation, any material and amount may be used as needed, without departure from the scope as defined by the following claims.

## Claims

1. A method for the cultivation of the microalgae of the genus Chlorella Vulgaris without genetic modification comprising:
- cultivating a mass of said microalgae in a photo-bioreactor;
**Characterized in that** said cultivating comprises:
- exposing said microalgae to stress conditions by ultrasonic technique, to obtain a stressed microalgae, said stress conditions comprising:
- immersing said microalgae in a tank surrounded by low-frequency ultrasonic transducers with adjustable frequency and power and containing a water-soluble conductive gel deprived of nitrogenated nutrients or deprived of phosphorus, whereby reduction of the thickness of the chloroplast of the stressed microalgae with respect of the thickness of the chloroplast of a Chlorella Vulgaris is obtained, so as to avoid absorbing of excess of light and whereby contemporarily increasing of the volume of the cytoplasm of the stressed microalgae with respect of the volume of the cytoplasm of a Chlorella Vulgaris is obtained, so as to storage an increased amount of lipids.

2. The method of claim 1, wherein said cultivation comprises a phototrophic cultivation using carbon dioxide derived from combustion, engine or methane furnace fumes and by an heterotrophic fermentation and wherein said phototrophic cultivation consists in cultivating said microalgae by the sunlight and well water.

3. The method as claimed in claim 2, wherein it comprises light aliphatic hydrocarbons from said microalgae.

4. The method as claimed in claim 3, wherein said light aliphatic hydrocarbons comprise between sixteen and twenty-six carbon atoms.

5. The method as claimed in claim 2, wherein said heterotrophic cultivation consists in feeding said microalgae with glucose.

## Patentansprüche

1. Verfahren zur Kultivierung von Mikroalgen der Gattung Chlorella Vulgaris ohne genetische Veränderung, umfassend:
- Kultivieren einer Masse der Mikroalgen in einem Photobioreaktor;
**dadurch gekennzeichnet, dass** das Kultivieren umfasst:
- Aussetzen der Mikroalgen Stressbedingungen durch Ultraschalltechnik, um eine gestresste Mikroalge zu erhalten, wobei die Stressbedingungen umfassen:
- Eintauchen der Mikroalgen in einen Tank, der von niederfrequenten Ultraschallwandlern mit einstellbarer Frequenz und Leistung umgeben ist und ein wasserlösliches leitfähiges Gel enthält, dem stickstoffhaltige Nährstoffe entzogen sind oder dem Phosphor entzogen ist, wodurch die Verringerung der Dicke des Chloroplasten der gestressten Mikroalgen in Bezug auf die Dicke des Chloroplasten einer Chlorella Vulgaris erhalten wird, um die Absorption von Lichtüberschuss zu vermeiden und wodurch gleichzeitig das Erhöhen des Volumens des Zytoplasmas der gestressten Mikroalgen in Bezug auf das Volumen des Zytoplasmas einer Chlorella Vulgaris erhalten wird, um eine erhöhte Menge an Lipiden zu speichern.

2. Verfahren nach Anspruch 1, wobei die Kultivierung eine phototrophe Kultivierung unter Verwendung von Kohlendioxid umfasst, das aus Verbrennungs-, Motor- oder Methanofendämpfen und durch eine heterotrophe Fermentation abgeleitet wird, und wobei die phototrophe Kultivierung darin besteht, die Mikroalgen durch Sonnenlicht und Brunnenwasser zu kultivieren.

3. Verfahren nach Anspruch 2, das leichte aliphatische Kohlenwasserstoffe aus den Mikroalgen umfasst.

4. Verfahren nach Anspruch 3, wobei die leichten aliphatischen Kohlenwasserstoffe zwischen sechszehn und sechsundzwanzig Kohlenstoffatomen umfassen.

5. Verfahren nach Anspruch 2, wobei die heterotrophe Kultivierung im Füttern der Mikroalgen mit Glucose besteht.

## Revendications

1. Procédé pour la culture de la microalgue du genre Chlorella vulgaris sans modification génétique comprenant :
- la culture d'une masse de ladite microalgue dans un photo-bioréacteur ;
**caractérisé en ce que** ladite culture comprend :
- l'exposition de ladite microalgue à des conditions de stress par une technique ultrasonore, pour obtenir une microalgue stressée, lesdites conditions de stress comprenant :
- l'immersion de ladite microalgue dans une cuve entourée par des transducteurs ultrasonores à basse fréquence ayant une fréquence et une puissance réglables et contenant un gel conducteur hydrosoluble dépourvu de nutriments azotés ou dépourvu de phosphore, selon laquelle une réduction de l'épaisseur du chloroplaste de la microalgue stressée par rapport à l'épaisseur du chloroplaste de Chlorella vulgaris est obtenue, afin d'éviter l'absorption d'un excès de lumière et selon laquelle une augmentation simultanée du volume du cytoplasme dans la microalgue stressée par rapport au volume du cytoplasme d'une Chlorella vulgaris est obtenue, enfin de stocker une plus grande quantité de lipides.

2. Procédé selon la revendication 1, dans lequel ladite culture comprend une culture phototrophe utilisant du dioxyde de carbone dérivé d'une combustion, d'un moteur ou de fumées de four de méthane et par une fermentation hétérotrophe et dans lequel ladite culture phototrophe consiste en la culture de ladite microalgue par la lumière solaire et une eau de puits.

3. Procédé selon la revendication 2, dans lequel il comprend des hydrocarbures aliphatiques légers provenant de ladite microalgue.

4. Procédé selon la revendication 3, dans lequel lesdits hydrocarbures aliphatiques légers comprennent entre seize et vingt-six atomes de carbone.

5. Procédé selon la revendication 2, dans lequel ladite culture hétérotrophe consiste en l'alimentation de ladite microalgue avec du glucose.
